Europäisches Patentamt

European Patent Office

Office européen des brevets

(19)

(11) **EP 1 174 132 A1**

(12) **EUROPEAN PATENT APPLICATION**
published in accordance with Art. 158(3) EPC

(43) Date of publication:
**23.01.2002 Bulletin 2002/04**

(51) Int Cl.[7]: **A61K 31/216**, A61K 47/32,
**A61K 9/70**, A61P 31/02

(21) Application number: **00917457.4**

(22) Date of filing: **25.04.2000**

(86) International application number:
**PCT/JP00/02697**

(87) International publication number:
**WO 00/64435 (02.11.2000 Gazette 2000/44)**

(84) Designated Contracting States:
**AT BE CH CY DE DK ES FI FR GB GR IE IT LI LU MC NL PT SE**
Designated Extension States:
**AL LT LV MK RO SI**

(30) Priority: **26.04.1999 WOPCT/JP02/21399**

(71) Applicant: **Lead Chemical Co. Ltd.**
**Toyama-shi, Toyama-ken 930-0912 (JP)**

(72) Inventors:
• **MORI, Jun**
  **Toyama-shi, Toyama 930-0952 (JP)**

• **YAMA, Seijiro**
  **Toyama-shi, Toyama 930-0928 (JP)**
• **HAMAMOTO, Tetsukazu**
  **Toyama-shi, Toyama 939-8015 (JP)**
• **TAKAO, Sunao**
  **Oyama-machi, Kaminiikawa-gun, Toyama 930 (JP)**
• **WAKI, Hitomi**
  **Fuchu-machi, Nei-gun, Toyama 939-2723 (JP)**

(74) Representative: **Müller - Hoffmann & Partner**
**Patentanwälte P.O. Box 80 12 20**
**81612 München (DE)**

(54) **PERCUTANEOUS ABSORPTION PREPARATIONS CONTAINING OXYBUTYNIN**

(57)    The present invention relates to transdermal absorption preparations comprising oxybutynin or its pharmacologically acceptable salt thereof as the active ingredient, and N-vinyl acetamide copolymer as the main component, containing a polymerizable monomer having N-vinyl acetamide and a COOM group wherein M represents hydrogen, an alkali metal or ammonium. The present invention further relates to non-aqueous transdermal absorption preparations containing, as an-other base component, a stabilizer comprising a cross-linking agent, polyhydric alcohols and a pH regulating agent, optionally together with a percutaneous absorption accelerator and/or solvent. These preparations may be an aqueous preparations which further contain an aqueous polymeric compound and water. These preparations are excellent in the percutaneous abosorbability of the active ingredient and drug stability, show little skin irritation and exhibit an excellent drug effect.

EP 1 174 132 A1

**Description**

Technical field

The present invention relates to transdermal absorption preparations used as the treating drugs for bladder functional disorder such as urinary incontinence, neurogenic urinary urgency or pollakiuria. In more detail, the present invention relates to transdermal absorption preparations for the treatment of bladder functional disorder comprising oxybutynin (chemical name: 4-diethylamino-2-butynyl(±)-α-cyclohexyl-α-phenyl glycolate) or its pharmacologically acceptable salt thereof as the main component.

Background art

In recent years, the countermeasure for the bladder functional disorder such as urinary incontinence or pollakiuria associated with the increase in the population of the advanced age group is one of the most important question of vital interest in the medical treatment. Therefore, the development of the effective drugs in treating urinary incontinence or pollakiuria are to be desired, and various medicines in addition to oral drugs already available in the market are on their way to development.

Oxybutynin hydrochloride used in the treatment of urinary incontinence and pollakiuria is well recognized as the excellent anticholinergic drug demonstrating its pharmacological effect through acetylcholine antagonism. An oral dosage form of the drug requires a comparatively small quantity of 2∼3 mg per dose, but they have to be taken three times a day. In addition, the absorption of the drug through the intestinal tract is known to be good, but the higher hepatic metabolism is also reported (Pharmacopoeia 4 (5), 45-53, 1990).

Regarding the routes of administration, the oral form has the advantage in not giving pain to patients as compared with the injection form, but it may not be easy to administer the medicine which has to be taken at the regular interval for the aged patients who may sometimes require the medical helper. Furthermore, the drug taken orally is inevitably absorbed into a hepatoportal vein through the intestinal tract, thereby being subjected to the first pass effect termed for the intense hepatic metabolism of the drug on its first passage and often leads to the marked decrease in biological availability in many cases. In order to maintain the effective concentration of the drug in the blood, it is necessary to administer a relatively large dose of drug, and as a result, an incidence in adverse effects naturally increases. From these standpoints, there is the urgent need for the development, of a preparation that is relatively easy to administer, long lasting in its effect, and yet with fewer adverse effects.

In view of pharmacokinetics, a preparation that does not exhibit the behavior of a transitory drug concentration in the blood such that the blood concentration rapidly increases and then decreases as often observed in the general orally administrated preparation, but whose concentration increases gradually and its effective concentration in the blood can be continuously maintained over a long period of time is highly desired.

On the other hand, the medical plaster-type transdermal absorption preparations for the external application has the following advantages:

a) Administration and interruption thereof of the drug are easy, and in the event any adverse effect is observed, administration of the drug can be interrupted by merely removing the preparation;
b) It is possible to continuously maintain the effective drug concentration in the blood over a long period of time;
c) Due to the advantage b) above, it is possible to decrease the frequency of administration, leading to the improved drug compliance by the patients, and the decreased responsibility for the helper;
d) Adverse effects can be avoided with the maximum blood concentration being lower than the maximum blood concentration observed for the oral administration; and
e) The undesirable first pass effect through the hepatoportal system can be avoided, and thus the biological availability of the drug can be enhanced.

Accordingly, the instant inventors have examined the transdermal absorption preparations comprising oxybutynin with the various advantages listed above in place of the oral administration as the alternative method of administration.

In recent years, a number of inventors disclosed the medical plaster-type preparations for the external application comprising oxybutynin (JP Laid-open Hei 4-266821, JP Laid-open Hei 4-273818, JP Laid-open Hei 6-145052 and JP National Publication Hei 10-507199).

However, the scientific evidences were not sufficiently disclosed in these laid-open specifications described above on the drug stability in the preparation, the skin irritability with the preparation, attaining and maintaining the effective blood concentration after the application of the preparation on the skin, and the pharmacological effect. For example, JP Laid-open Hei 4-266821 and JP Laid-open Hei 4-273818 have disclosed the transdermal absorption preparations containing oxybutynin, but they do not contain any concrete disclosures on the drug stability in the preparation, the skin irritability with the preparation, and the pharmacological effect. JP Laid-open Hei 6-145052 disclosed the percutaneous preparation with a free form of oxybutynin used as a raw drug and the rubbery base, but they only disclosed the drug stability in said rubbery base and the blood concentration measurement after their application on the skin of rats in the embodiments without the disclosure of the pharmacological effect was not disclosed. JP National Publication Hei 10-507199 disclosed the preparation containing a free form of oxybutynin as the raw drug upon consideration of the base and the percutaneous absorption accelerator therewith in order to attain the sufficient transdermal absorption with the scientific knowledge that oxybutynin was a basic drug. However, the drug stability in the preparation, the skin irritability, and the pharmacological effect were not disclosed in the publication.

Further, the medical plaster-type transdermal absorption preparations are generally classified as a tape preparation with the main base of a rubbery polymeric compound used as an adhesive, and as a poultice with the main base of an aqueous polymeric compound. The tape preparation with the main base of a rubbery polymeric compound as an adhesive has the excellent adhesive property to the skin, but their high skin irritability occasionally produce undesirable side effects such as rash or erythema. Therefore, this preparation is not suitable for the patient who required the long term repeated drug administration in chronic diseases. Further, when the drug has a salt bond, it may be difficult to form a preparation because of a limit in the selection of the solvent. On the other hand, since the poultice with the main base of an aqueous polymeric compound has relatively low skin irritability, and thus is suitable for long term repeated administration. However, when an ionic aqueous polymeric compound is used as the base, aluminum cross-linking produces the adhesive property as well as the form-keeping property, but the releasing property of the drug from the preparation decreases as the result of the basic compound such as oxybutynin forming an ionic complex with the anionic component of the base in the preparation.

The objects of the present invention are to iron out any difficulties in the existing arts, and to meet the necessary requisites such as the drug stability in the preparation, the low irritability to the skin, and attaining and maintaining the effective blood concentration after application of the preparation on the skin, and to provide an excellent transdermal absorption preparation containing oxybutynin with the desirable pharmacological effect.

Disclosure of the Invention

The objective of the present invention is to provide the transdermal absorption preparations containing oxybutynin surpassing in the drug stability in the preparation, drug properties and the low skin irritability of the preparation, attaining and maintaining the effective blood concentration of the drug after application of the preparation on the skin, and further having sufficient pharmacological effect. The inventors of the present invention were successful in preparing the transdermal absorption preparation containing oxybutynin with N-vinyl acetamide copolymer as the main base after the energetic investigation in order to achieve the final objectives described above.

That is to say that the present invention relates to the transdermal absorption preparation comprising the main base of N-vinyl acetamide copolymer comprising a polymeric monomer having N-vinyl acetamide and COOM group, wherein M is hydrogen, an alkali metal or ammonium as the main component, compounded therein oxybutynin or its pharmacologically acceptable salt thereof as the active ingredient.

N-vinyl acetamide copolymer is characterized with the water-soluble polymeric compound that can be used for both the non-aqueous transdermal absorption preparations and the aqueous transdermal absorption preparation, and furthermore, is a gel with good adhesive property, is soft on the skin contact in comparison with a rubbery polymeric compound, and has considerably low skin irritability. In addition, N-vinyl acetamide is non-ionic such that the ionic interaction with the basic compound such as oxybutynin in the preparation does not take place. Therefore, the releasing property of the drug from the preparation is not affected (JP Laid-open Hei 4-182437).

The examples of the polymeric monomer having COOM group copolymerized with N-vinyl acetamide compounded in the transdermal absorption preparation of the present invention are polymeric organic acids such as itaconic acid, maleic acid, maleic acid anhydride, crotonic acid and (meth)acrylic acid or their salts thereof, but (meth)acrylic acid or their salts (for example, sodium salt, potassium salt, ammonium salt) is preferred. The weight ratio of N-vinyl acetamide and the copolymerized monomer is preferably in the range of 99.9:0.1 to 60:40. N-vinyl acetamide copolymer used as the preparation base is, for example, PNVA, a product of Showa Denko K.K., especially PNVA GE-167. The compounding amount of N-vinyl acetamide copolymer or its cross-linked product is preferably 2% to 50% by weight, and more preferably 5% to 25% by weight, based on the total weight of the preparation.

Oxybutynin which is the active ingredient in the preparation of the present invention may be free, and further may be inorganic acid addition salts such as hydrochloride, sulfate, hydrobromide, nitride or phosphate, or organic acid addition salts such as acetate, succinate, fumarate, malate or oxalate. In the preparation of the present invention, oxybutynin or pharmacologically acceptable salt thereof which is the active ingredient are preferably compounded in the amount of 0.01% to 30% by weight, more preferably about 0.1% to 10% by weight, and the most preferably about 0.5% to 5% by weight, based on the total weight of the preparation, although the amount to some extent varies according to the preparation form or the base components.

The transdermal absorption preparation of the present invention may contain, in addition to oxybutynin and N-vinyl acetamide copolymer, various bases customarily used for the external preparation as long as they do not affect the other components, such as cross-linking agent, di- or polyhydric alcohols that can be used as the thickening agent and/or the moisturizer, aqueous polymeric compounds that can be used as the pressure-sensitive adhesive and/or form-keeping agent, liposoluble polymeric compounds that can be used as the adhesive and/or tackifier, percutaneous absorption accelerators, solvents, surfactants, stabilization agents, and other pharmacologically acceptable additives without any limitation.

Depending on the main base components, the transdermal absorption preparations can be classified as the non-aqueous transdermal absorption preparation comprising non-aqueous base components in addition to N-vinyl acetamide copolymer as the main base, and as the aqueous transdermal absorption preparation comprising aqueous base components in addition to N-vinyl acetamide copolymer as the main base.

Therefore, one of the preferred embodiment for the transdermal absorption preparation of the present invention comprises N-vinyl acetamide copolymer comprising, as the main component, a polymeric monomer having N-vinyl acetamide and COOM group, wherein M is hydrogen, an alkali metal or ammonium, an organic or an inorganic compound forming divalent or trivalent metal ions as the cross-linking agent, di- or polyhydric alcohols as the thickening agent and/or the moisturizer, stabilization agent, especially pH regulating agent as the base components, and further is the non-aqueous transdermal absorption preparation base containing oxybutynin or pharmacologically acceptable salt thereof as the active ingredient. It is preferred that the preparation additionally contains the percutaneous absorption accelerator or solvent, or both percutaneous absorption accelerator and solvent.

Further, one of the other preferred embodiment for the transdermal absorption preparation of the present invention comprises N-vinyl acetamide copolymer comprising, as the main component, a polymeric monomer having N-vinyl acetamide and COOM group, wherein M is hydrogen, an alkali metal or ammonium, an organic or an inorganic compound forming divalent or trivalent metal ions as the cross-linking agent, di- or polyhydric alcohols as the thickening agent and/or the moisturizer, water, the aqueous polymeric compound, the stabilization agent, and especially pH regulating agent as the base components, and further is the aqueous transdermal absorption preparation containing oxybutynin or pharmacologically acceptable salt thereof as the active ingredient. It is preferred that the preparation additionally contains the percutaneous absorption accelerator or the solvent other than water, or both the percutaneous absorption accelerator and the solvent other than water.

The base components other than N-vinyl acetamide copolymer are described below.

The cross-linking agent for N-vinyl acetamide copolymer of the present invention is preferably the cross-linking agent such as salts that form divalent or trivalent metal ions. The amount to be compounded into the preparation is in the range of 0.01% to 20% by weight, and more preferably 0.1% to 10% by weight, based on the total weight of the preparation. Examples of the cross-linking agent that form divalent or trivalent metal ions include hydroxides such as aluminum hydroxide or magnesium aluminum hydroxide, inorganic or organic normal salts such as aluminum chloride, aluminum sulfate, dihyroxyaluminum aminoacetate, synthetic aluminum silicate, kaolin, aluminum stearate, magnesium hydroxide or magnesium sulfate, or their basic salts thereof, complex salts such as aluminum alum, aluminates such as sodium aluminate, inorganic aluminum complex salts, and organic aluminum chelate compounds, and they further include synthetic hydrotarcite, magnesium methasilicic acid aluminate, magnesium silicic acid aluminate, aluminum nitrate, aluminum sulfate, EDTA-aluminum, aluminum allantoinate, aluminum acetate, and aluminum glycinal among others, but examples are not limited to those described above. Further, one kind or more than two kinds of these salts that form divalent or trivalent metal ions can be used. The salts that form divalent or trivalent metal ions may be water-soluble or sparingly water-soluble. When the sparingly water-soluble aluminum compound is used, the rate modifier can be added in the reaction system for the gelation, and in particular, the reaction rate for gelation is accelerated with the addition of an acid. The reaction becomes markedly rapid with the addition of particularly an organic acid with hydroxyl group or its salts as the acid. Examples of the rate modifier for the cross-linking reaction that can be used include citric acid, lactic acid, tartaric acid, gluconic acid, glycolic acid, malic acid, fumaric acid, metasulfonic acid, maleic acid, acetic acid, EDTA-2 sodium, urea, triethyl amine, an organic acid having chelating or coordination ability to metal ion such as ammonia, organic acid salts, organic salt groups, and inorganic acids such as hydrochloric acid, phosphoric acid, sulfuric acid or hydrobromic acid among others, but examples are not limited to those described above.

Examples of di- or polyhydric alcohols that can be used as the thickening agent and/or the moisturizer, include ethylene glycol, propylene glycol, trimethylene glycol, 1,3-butanediol, ethylene glycol monobutyl ether, triethylene glycol, 1,4-butanediol, glycerin, trioxyisobutane, erythritol, pentaerythritol, xylitol, adonitol, allodulcitol, D-sorbitol, liquid sorbitol, mannitol and polyethylene glycol among others. In particular, polyethylene glycol, 1,3-butanediol, propylene glycol and glycerin are preferred and one kind or more than two kinds of these polyhydric alcohols can be conveniently mixed for the use. The amount of these polyhydric alcohols to be compounded is preferably 10% to 95% by weight, and more preferably 20% to 70% by weight, based on the total weight of the preparation.

These polyhydric alcohols can be used as the solvent or the percutaneous absorption accelerator, but ethylene glycol, propylene glycol and 1,3-butanediol were found to be particularly suited as the percutaneous absorption accelerator.

Other percutaneous absorption accelerators are not particularly limited so long as they can be generally used in the transdermal absorption preparation, and such examples include aliphatic alcohols such as ethanol, lauryl alcohol, myristyl alcohol, cetyl alcohol and oleyl alcohol, fatty acids such as lauric acid, myristic acid, palmitic acid, oleic acid, stearic acid, ethyl lactate, cetyl lactate, isopropyl myristic acid, isopropyl palmitic acid, oleyl oleic acid, diisopropyl adipic acid, diisopropyl sebacic acid, glycerine monocapric acid and ethyleneglycol monoisooctanoic acid or fatty acid esters, oils of animal and plant origin such as almond oil, olive oil, camellia oil, persic oil, peppermint oil, soybean oil, sesame oil, mink oil, cottonseed oil, palm oil, castor oil and eucalyptus oil, terpene compounds such as I-menthol, I-menthone, limonene, pinene, terpinene, terpinol, carveol and dl-camphor, pyrrolidone derivatives such as N-methyl pyrrolidone, especially N-methyl-2-pyrrolidone, ureas, crotamiton, benzyl alcohol, cyclodextrin, calcium thioglycotic acid, sodium capric acid, volatile oil, other hydrocarbons, propylene carbonate, Eizon (chemical name: 1-dodecylazacycloheptan-2-one), other organic acids, and organic acid esters among others. One kind or more than two kinds of these components can be used, and the amount of the percutaneous absorption accelerator to be compounded into the preparation is preferably in the range of 0.5% to 20% by weight, more preferably 1% to 15% by weight, based on the total weight of the preparation. Aliphatic alcohols, fatty acids and their esters, and oils of animal and plant origin described above can be used as the solvent as well. The preferred percutaneous absorption accelerator in the non-aqueous transdermal absorption preparation is propylene glycol, Crotamiton, N-methyl-2-pyrrolidone, I-menthol, lactic acid esters and fatty acid esters, and their combination thereof. On the other hand, the preferred percutaneous absorption accelerator in the aqueous transdermal absorption preparation is cetyl lactate, oleyl alcohol, Crotamiton, isopropanol, N-methyl-2-pyrrolidone and EDTA and their combination thereof, and particularly preferred is cetyl lactate and oleyl alcohol.

The preferred solvents for the transdermal absorption preparation of the present invention is, for example, water, methanol, ethanol, n-propanol, isopropanol, acetone and their combination thereof, and they can be added based on the proper ratio within the range that do not affect the production of the preparation. In particular, the amount of water to be compounded in the aqueous system preparation obtained by using water as solvent is preferably in the range of 5% to 90% by weight, more preferably 10% to 70% by weight, based on the total weight of the preparation.

Aqueous polymeric compounds that can be used as pressure-sensitive adhesive and/or form-keeping agent are, for example, natural polymers such as gum arabic, tragacanth gum, locust bean gum, guar gum, ECHO GUM (xanthan gum), karaya gum, agar-agar, starch, carrageenan, alginic acid, alginate (such as sodium alginate), propylene glycol alginate, dextran, dextrin, amylose, gelatin, collagen, pullulan, pectin, amylopectin, starch, sodium amylopectin hemiglycolate, chitin, albumin, casein or crystaline cellulose; semi-synthetic polymeric compounds such as polyglutamic acid, polyasparagic acid, methyl cellulose, ethyl cellulose, propyl cellulose, ethyl methyl cellulose, hydroxymethyl cellulose, hydroxyethyl cellulose, hydroxypropylmethyl cellulose, hydroxypropyl starch, carboxymethyl starch, alkali metal carboxymethyl cellulose, alkali metal cellulose sulfate, cellulose graft polymer, cross-linked gelatin, starch-acrylic acid graft polymer, phthalic anhydride-modified gelatin or succinic acid-modified gelatin; and synthetic polymeric compounds such as polyvinyl alcohol, polyvinyl pyrrolidone, polyvinyl methylether, polyacrylic acid, polyacrylic acid salts (such as sodium polyacrylate), starch acrylate, carboxyvinyl polymer, polyvinyl sulfonic acid, polyitaconic acid, polyhydroxyethyl acrylate, polyacrylamide, acrylate ester copolymer and its emulsion, methylvinyl ether/malic anhydride copolymer and its emulsion, vinyl pyrrolidone-ethyl acrylate copolymer, vinyl pyrrolidone-styrene copolymer, vinyl pyrrolidone-vinyl acetate copolymer, polyvinyl acetate-(meth)acrylic acid copolymer, polyvinyl acetate-crotonic acid copolymer, vinylacetate-(meth)acrylic acid copolymer, vinyl acetate-crotonic acid copolymer, styrene-maleic anhydride copolymer, or acrylamide-acrylic acid copolymer; but not necessary limited to these compounds. Preferred aqueous polymeric compounds are polyacrylate, methyl cellulose, starch acrylate, and acrylic acid ester copolymer and its emulsion. One-kind or more than two kinds of these aqueous polymeric compounds can be combined, and the amount to be compounded into the preparation is in the range of 3% to 70% by weight, more preferably 5% to 50% by weight, based on the total weight of the preparation.

Examples of liposoluble polymeric compounds which can be used as adhesive and/or tackifier are natural rubber, isoprene rubber, polyisobutylene rubber, styrene butadiene rubber, styrene isoprene styrene block copolymer, styrene butadiene styrene block copolymer, (meth)acrylic acid ester copolymer, silicone resin, rosin, ester gum, polybutene, lanoline, vaseline, Plastibase, beeswax, paraffin, liquid paraffine, and squalane. One kind or more than two kinds of these compounds can be compounded, and the amount to be compounded into the preparation is preferably in the range of 5% to 80% by weight, more preferably 10% to 50% by weight, based on the total weight of the preparation.

General pH regulating agent can be used as the stabilizer. Examples thereof include inorganic acids such as hydrochloric acid, sulfuric acid, hydrobromic acid, phosphoric acid and boric acid, or their salts, organic acids such as acetic acid, succinic acid, fumaric acid, malic acid, oxalic acid, lactic acid, glutaric acid, salicylic acid, citric acid and tartaric acid, or their salts, fatty acids such as palmitic acid, stearic acid, oleic acid and linoleic acid, or their salts, inorganic bases such as sodium hydroxide and calcium hydroxide, and organic bases such as ammonia, diisopropanol amine, diethanol amine, triethanol amine and triethylamine. Examples of preferred pH regulating agents are phosphoric acid, boric acid, acetic acid, tartaric acid, lactic acid and citric acid, and their salts. Antioxidants can be further used as the stabilizers, and such examples include sodium hydrogen sulfite, L-ascorbic acid, sodium ascorbate, butylhydroxyanisole, dibutylhydroxytoluene, propyl gallate, tocopherol acetate, and D-$\alpha$-tocopherol. The amount of these to be compounded is 0.01 to 10% by weight, and preferably 0.1 to 5% by weight, based on the total weight of the preparation. Other stabilizers are surfactant and inorganic substance. Examples of surfactant that can be optionally added are sorbitan monooleic acid, polyoxyethylene sorbitan monooleic acid and polyoxyethylene laurylether among others, and examples of inorganic substances are bentonite, kaolin, talc, white carbon (hydrous silicon dioxide), titanium white and calcium carbonate. The amount of these to be compounded is in the range of 0.01 to 15% by weight, and more preferably 0.1 to 10% by weight, based on the total weight of the preparation. One kind or the combination of more than two kinds of these stabilizers can be added, and they can be compounded with an appropriate quantitative ratio as long as they are within this range.

Other pharmacologically acceptable additives, antiseptics (preservatives) such as para-hydroxy methyl benzoate, para-hydroxy ethyl benzoate, para-hydroxy propyl benzoate, chlorobutanol and benzyl alcohol, and perfumes among others can be compounded. The amount to be compounded is preferably in the range of 0.01% to 15% by weight, and more preferably 0.1% to 10% by weight.

Adhesive imparting agents, softening agents, age resisters, fillers among others which are commonly used in the transdermal absorption preparation currently on the market can be compounded into the transdermal absorption preparation of the present invention at need in addition to each component listed above. Furthermore, they can contain all kinds of emulsifier, dispersant, wetting agent, stabilizer other than one listed above and suspending agent among others.

Followings are examples of preferred compositions for the non-aqueous transdermal absorption preparation containing oxybutynin of the present invention. Each composition for the preparation is compounded so as to collectively make 100% by weight. Preferred compositions are:

(1) The transdermal absorption preparation with oxybutynin containing at least 0.01% to 30% by weight of oxybutynin hydrochloride, 2% to 50 % by weight of N-vinyl acetamide copolymer, 0.01% to 20% by weight of a cross-linking agent, 10% to 90% by weight of di- or polyhydric alcohols, 0.5% to 20% by weight of a percutaneous absorption accelerator or a solvent or both percutaneous absorption accelerator and solvent, and 0.01% to 10% by weight of pH regulating agent, and in addition optionally 5% to 80% by weight of liposoluble polymeric compounds and additionally 0.01% to 15% by weight of other bases if necessary, based on the total weight of the preparation.

It is more preferably:

(2) The transdermal absorption preparation with oxybutynin containing at least 0.1% to 10% by weith of oxybutynin hydrochloride, 5% to 25% by weight of N-vinyl acetamide copolymer, 0.1% to 10% by weight of a cross-linking agent, 20% to 70% by weight of di- or polyhydric alcohols, 1% to 25% by weight of a percutaneous absorption accelerator or a solvent, or both percutaneous absorption accelerator and solvent, 0.1% to 5% by weight of pH regulating agent, optionally 10% to 50% by weight of liposoluble polymer, and furthermore, 0.1% to 10% by weight of other bases if necessary, based on the total weight of the preparation.

It is more preferably:

(3) The transdermal absorption preparation with oxybutynine containing at least 0.5% to 5% by weight of oxybutynin hydrochloride, 5% to 25% by weight of N-vinyl acetamide copolymer, 0.1% to 10% by weight of a cross-linking agent, 20% to 70% by weight of di- or polyhydric alcohols, 1% to 15% by weight of a percutaneous absorption accelerator or a solvent, or both percutaneous absorption accelerator and solvent, 0.1% to 5% by weight of pH regulating agent, optionally 10% to 50% by weight of liposoluble polymeric compounds, and additionally 0.1% to 10% by weight of other bases if necessary, based on the total weight of the preparation.

(4) In case di- or polyhydric alcohols such as ethylene glycol, propylene glycol or 1,3-butane diol can be used as the percutaneous absorption accelerator and the solvent described above, the transdermal absorption preparation with oxybutynin containing at least 0.01% to 30% by weight of oxybutynin hydrochloride, 2% to 50% by weight of N-vinyl acetamide copolymer, 0.01% to 20% by weight of a cross-linking agent, 10% to 90% by weight of di- or polyhydric alcohols, and 0.01% to 10% by weight of pH regulating agent, and optionally 5% to 80% by weight of liposoluble polymeric compounds, and additionally 0.01% to 15% by weight of other bases if necessary, based on the total weight of the preparation.

Furthermore, examples of preferred composition for the aqueous transdermal absorption preparation containing oxybutynin of the present invention are described below. Each composition for the preparation is compounded so as to collectively make 100% by weight. Preferred compositions are:

(1) The transdermal absorption preparation with oxybutynin containing at least 0.01% to 30% by weight of oxybutynin hydrochloride, 2% to 50% by weight of N-vinyl acetamide copolymer, 0.01% to 20% by weight of a cross-linking agent, 10% to 90% by weight of di- or polyhydric alcohols, 0.5% to 20% by weight of a percutaneous absorption accelerator or a solvent other than water, or both percutaneous absorption accelerator and solvent other than water, 5% to 90% by weight of water, 3% to 70% by weight of aqueous polymeric compounds, 0.01% to 10% by weight of pH regulating agent, optionally 5% to 80% by weight of liposoluble polymeric compounds, and additionally 0.01% to 15% by weight of other bases if necessary, based on the total weight of the preparation.

It is more preferrably:

(2) The transdermal absorption preparation with oxybutynin containing at least 0.1% to 10% by weight of oxybutynin hydrochloride, 5% to 25% by weight of N-vinyl acetamide copolymer, 0.1% to 10% by weight of a cross-linking agent, 20% to 70% by weight of di- or polyhydric alcohols, 1% to 15% by weight of a percutaneous absorption accelerator or a solvent other than water, or both percutaneous absorption accelerator and solvent other than water, 10% to 70% by weight of water, 5% to 50% by weight of aqueous polymeric compounds, 0.1% to 5% by weight of pH regulating agent, optionally 10% to 50% by weight of liposoluble polymeric compounds, and additionally 0.1% to 10% by weight of other bases if necessary, based on the total weight of the preparation.

It is more preferred:

(3) The transdermal absorption preparation with oxybutynin containing at least 0.5% to 5% by weight of oxybutynin hydrochloride, 5% to 25% by weight of N-vinyl acetamide copolymer, 0.1% to 10% by weight of a cross-linking agent, 20% to 70% by weight of di- or polyhydric alcohols, 1% to 15% by weight of a percutaneous absorption accelerator or a solvent other than water, or both percutaneous absorption accelerator and solvent other than water, 10% to 70% by weight of water, 5% to 50% by weight of aqueous polymeric compounds, 0.1% to 5% by weight of pH regulating agent, optionally 10% to 50% by weight of liposoluble polymeric compounds, and additionally 0.1% to 10% by weight of other bases if necessary, based on the total weight of the preparation.

The transdermal absorption preparations of the present invention are mainly in the forms of medical plaster-type such as poultice or plaster as the external application preparation or can take the other forms such as ointment, cream, liquid solution, emulsion or suspension, but the preferred is medical plaster-type.

The transdermal absorption preparation containing oxybutynin of the present invention as the medical plaster-type can as a general rule be prepared as follows.

That is, the plaster containing oxybutynin is at first prepared by dissolving oxybutynin or its pharmacologically acceptable salt thereof as the active ingredient into the solvent, and then adding N-vinyl acetamide copolymer and optionally varieties of bases normally used for any transdermal absorption preparation described above if necessary. Next, the plaster thus prepared is spread out over the appropriate support and then the strippable film is adhered to the exposed surface of the plaster opposite to the support side, or conversely the plaster is spread out over the strippable film and the support is adhered to the exposed surface of the plaster opposite to the strippable film side, and thus the transdermal absorption medical plaster-type preparation of the present invention can be provided after cutting them into an appropriate size.

Any support materials and any optional support materials used in the past as well can be used without limitation for the transdermal absorption preparations described above, but preferred examples are such as flexible film made of vinyl chloride, polyethylene type film, ethylene copolymer type film or polypropylene type film, and additionally cotton clothes or non-woven fabrics and their laminates with plastic films. Any optional films made of such as polyethylene, polypropylene or polyester that have been used in the past can be used as the strippable film.

Followings are concrete examples of the production of the transdermal absorption preparations of the present invention as the medical plaster.

Non-aqueous type preparation

0.5% to 5% by weight of oxybutynin hydrochloride is dissolved in isopropanol equal to or 1.5 times of the total weight of the preparation, and further 5% to 25% by weight of N-vinyl acetamide copolymer, 0.1% to 10% by weight of cross-linking agent, stabilizers such as the suitable amount of pH regulating agent and other bases if necessary are added and stirrer-mixed. Then, the plaster is prepared by the addition of 20% to 70% by weight of glycerin and other polyhydric alcohol prepared in advance and the mixture solution containing 1% to 15% by weight of the suitable percutaneous absorption accelerator if necessary. The mixture is kept stirred until the desirable viscosity is attained for the application of the plaster, and then they are spread out over the support, solvent is removed by heat drying (solvent drying), the strippable film is adhered to the exposed surface of the plaster opposite to the support side, and finally the non-aqueous type transdermal absorption preparation of the object is prepared by the solvent method. In addition, when 10% to 50% by weight of liposoluble polymeric compounds are compounded into the preparation described above, said compounds are added into the oxybutynin hydrochloride solution.

Aqueous type preparation

(I) 0.5% to 5% by weight of oxybutynin hydrochloride is dissolved in isopropanol equal to or 1.5 times of the total weight of the preparation, and further 5% to 25% by weight of N-vinyl acetamide copolymer, 0.1% to 10% by weight of cross-linking agent, stabilizers such as the suitable amount of pH regulating agent and other bases if necessary, and also 5% to 50% by weight of aqueous polymeric compounds are added and stirrer-mixed. Then, the plaster is prepared by the addition of 20% to 70% by weight of glycerin or other polyhydric alcohol prepared in advance and 10% to 70% by weight of water, and the mixture solution containing 1 % to 15% by weight of the suitable percutaneous absorption accelerator if necessary. The mixture is kept stirred until the desirable viscosity is attained for the application of the plaster, and then they are spread out over the support, solvent is removed by heat drying (solvent drying), the strippable film is adhered to the exposed surface of the plaster opposite to the support side, and finally the aqueous type transdermal absorption preparation of the object is prepared by the solvent method. In addition, when 10% to 50% by weight of liposoluble polymeric compounds are compounded into the preparation described above, said compounds are added into the oxybutynin hydrochloride solution.

(II) 0.5% to 5% by weight of oxybutynin hydrochloride is dissolved in 10% to 70% by weight of water, and further 5% to 25% by weight of N-vinyl acetamide copolymer dispersed in 5% to 20% by weight of polyhydric alcohol such as plyethylene glycol in addition to suitable amount of pH regulating agent is added, then the mixture solution prepared in advance consisting of 20% to 70% by weight of glycerin prepared, 0.1% to 10% by weight of inorganic substances and 5% to 50% by weight of aqueous polymeric compounds is added, and stirred. Subsequently, 1% to 15% by weight of a percutaneous absorption accelerator or a solvent, or both percutaneous absorption accelerator and solvent, the suitable amount of surfactant and pharmacologically acceptable additives are added, and incorporated. Furthermore, water suspension with 0.1% to 10% by weight of cross-linking agent is added and thoroughly kneaded. The aqueous type transdermal absorption preparation of the object is prepared by spreading out the plaster thus obtained over the strippable film and adhering the support to the exposed surface of the plaster opposite to the strippable film side.

In addition, the preparation with less water content can be prepared by the addition of 10% to 50% by weight of liposoluble polymeric compounds to the plaster described above.

When the aqueous type preparation is produced, spreading the plaster over the support or the strippable film is difficult upon incorporation since N-vinyl acetamide copolymer has the tendency to aggregate with the addition of water. Therefore, the spreading capacity of the plaster can be improved by dispersing N-vinyl acetamide copolymer in isopropanol according to the solvent method (I) above, or by the improve dispersibility of N-vinyl acetamid coolymer with the addition of inorganic substances according to the method (II) of the preparation.

The following characteristics are notable for the transdermal absorption preparation of the present invention when used as the medical plaster-type:

1) The non-aqueous type transdermal absorption preparation produced by the solvent method described above shows pronounced adhesive property and becomes more stable, since the cross-linking agent and pH regulating agent in the preparation function with the moisture transpired from the skin upon dermal application.

In addition, pH of the aqueous type preparation is important for the drug stability in the preparation, and its limit is pH 3.5 to pH 7.0, more preferably pH 4.0 to pH 5.5, and the most preferably pH 4.5 to 5.1.

2) Furthermore, an aqueous polymeric compound, N-vinyl acetamide copolymer, which is the main base for the preparation, is good adhesive gel, and they are soft on touch in comparison with rubbery polymeric compounds and show very low skin irritability. At the same time, the releasing property of the drug is not affected since the ionic interaction between the base and oxybutynin as a basic compound does not take place. Therefore, the preparations of the present invention show better skin permeability in comparison to the preparations made of rubbery polymeric compound bases heretofore or the aqueous preparations with aqueous polymeric compound base other than N-vinyl acetamide copolymer as apparent from the embodiments described below.

3) Moreover, the preparations of the present invention can maintain the sufficiently effective concentration of the drug in the blood after the dermal application of the preparations and the concentration in the blood decreases immediately upon the discontinuation of the application as evident from animal experiments described below. Furthermore, their observed pharmacological effects after the application of the active drug component proves that they are obviously the transdermal absorption preparation.

Brief Description of the Drawings

Fig. 1 is a graph showing the cumulative amount of oxybutynin hydrochloride with time which penetrates through a rat skin from the transdermal absorption preparation containing oxybutynin as shown in Examples 1, 2, 3 and 4 and Comparative Examples 1 and 2 (Experiment 1).

Fig. 2 is a graph showing the result of a primary irritation test using the rabbit skin with the transdermal absorption preparation containing oxybutynin as shown in Examples 1 and 2 (Experiment 3).

Fig. 3 is a graph showing the change in the concentration of oxybutynin hydrochloride with time in the rabbit blood plasma by the application to the rabbits of the transdermal absorption preparation containing oxybutynin as shown in Examples 1 and 2 (Experiment 4).

Fig. 4 is a graph showing the influence of the transdermal absorption preparation containing oxybutynin as shown in Example 1 to the cystometrogram of a vigilant rabbit (Experiment 5).

Best Mode for Carrying Out the Invention

The usefulness of the present invention is described by referring to the following examples, comparative examples and experiments, but the invention is not limited thereto. In addition, "part" referred in the examples and comparative examples are all part by weight.

Example 1

1.0 part of oxybutynin hydrochloride was dissolved in 200.0 parts of isoprapanol as the solvent, and then 20.0 parts of N-vinyl acetamide copolymer (PNVA GE167, a product of Showa Denko K.K.), 1.0 part of synthetic aluminum silicate and 1.0 part of borax were added and stir-mixed. The mixture solution containing 62.0 parts of glycerin and 15.0 parts of propylene glycol were added and continuously stirred.

The solvent-type plaster with the desirable viscosity for the plaster is spread out over the non-woven fabric, then solvent is removed by heat drying (solvent drying) and the strippable film made of polyester was adhered. This was cut into the desirable size to obtain the transdermal absorption preparation containing oxybutynin hydrochloride.

Example 2

3.0 parts of oxybutynin hydrochloride were dissolved in 150.0 parts of isoprapanol as the solvent, and then 15.0 parts of N-vinyl acetamide copolymer (PNVA GE167, a product of Showa Denko K.K.), 2.0 parts of synthetic aluminum silicate and 1.0 part of tartaric acid were added and stir-mixed. The mixture solution containing 56.0 parts of glycerin and 20.0 parts of propylene glycol were added and continuously stirred.

The solvent-type plaster with the desirable viscosity for the plaster is spread out over the non-woven fabric, then solvent was removed by heat drying and the strippable film made of polyester is adhered. This was cut into the desirable size to obtain the transdermal absorption preparation containing oxybutynin hydrochloride.

Example 3

4.0 parts of oxybutynin hydrochloride were dissolved in 150.0 parts of isoprapanol as the solvent, and then 10.0 parts of N-vinyl acetamide copolymer (PNVA GE167, a product of Showa Denko K.K.), 1.5 parts of aluminum gel GLYCINAL, 0.5 part of tartaric acid, 5.0 parts of methyl cellulose and 10.0 parts of polyacrylic acid solution were mixed by stirring. The mixture solution containing 39.0 parts of glycerin, 10.0 parts of propylene glycol and 20.0 parts of water were added and continuously stirred. The solvent-type plaster with the desirable viscosity for the plaster is spread out over the non-woven fabric, then solvent is removed by heat drying and the strippable film made of polyester was adhered. This was cut into the desirable size to obtain the aqueous transdermal absorption preparation containing oxybutynin hydrochloride. The pH of the preparation was pH 4.5.

Example 4

2.0 parts of oxybutynin hydrochloride were dissolved in 32.4 parts of water, and then 1.0 part of tartaric acid, 10.0 parts of polyacrylic acid solution and 10.0 parts of acrylric acid ester copolymer emulsion were added and stir-mixed. The mixture solution containing 6.0 parts of N-vinyl acetamide copolymer (PNVA GE167, a product of Showa Denko K.K.) and 8.0 parts of polyethylene glycol prepared in advance, and the mixture solution containing18.0 parts of glycerin, 5.0 parts of methyl cellulose and 5.0 parts of white carbon (hydrous silicon dioxide) were added and continuously stirred. Next, 0.3 part of polyoxyethylene laurylether and 2.0 parts oleyl alcohol were added and mixed. Finally, 0.3 part of dried alluminum hydroxide gel and 2.0 parts of water were added and mixed. The plaster thus obtained was spread out over the non-woven fabric and the strippable film made of polyester was adhered. This was cut into the desirable size to obtain the aqueous transdermal absorption preparation containing oxybutynin hydrochloride. The pH of the preparation was pH 5.1.

Comparative example 1

18.2 parts of polybutene, 9.1 parts of polyisobutylene (trade name: VISTANEX MML-80), 21.2 parts of styrene-isobutylene-styrene block copolymer (trade name: KARIFLEX 400P), 0.2 part of dibutyl hydroxytoluene, 10.6 parts of liquid paraffin, 9.0 parts of aqueous polymeric compound (trade name: SUNWET IM1000MPS), 21.2 parts of rosin ester (trade name: ESTERGUM H), 4.5 parts of aqueous polymeric compound (trade name: HIVISWAKO 304), 5.0 parts of N-methyl-2-pyrrolidone and 1.0 part of oxybutynin hydrochloride were dissolved in isohexane, and the resulting solution was spread out over the support made of vinyl chloride, then solvent was removed by heat drying and the strippable film made of polyester was adhered. This was cut into the desirable size to obtain the transdermal absorption preparation containing oxybutynin hydrochloride.

Comparative example 2

1.0 Part of oxybutynin hydrochloride was dissolved in 37.96 parts of water, and 0.4 parts of polysorbate 80, 0.2 part of sorbitan sesquioleate, 2.0 parts of tartaric acid and 8.0 parts of polyacrylic acid solution were added and continuously mixed. Then, the mixture solution containing 5.0 parts of sodium polyacrylic acid, 5.0 parts of starch polyacrylic acid, 0.4 parts of titanium oxide and 30.0 parts of glyceline was stir-mixed. Next, 10.0 parts of acrylic acid ester copolymer emulsion and 0.04 part of dried aluminium hydroxide gel were added and mixed. The plaster thus obtained was spread out over the non-woven fabric and the strippable film made of polyester was adhered. This was cut into the desirable size to obtain the transdermal absorption preparation containing oxybutynin hydrochloride.

Experiment 1: *In vitro* skin penetration test

1) Test method

*In vitro* skin penetration test was conducted for each preparation described in Examples 1, 2, 3 and 4 as well as Comparative examples 1 and 2 according to the method described below.

The abdominal region of Wistar male rat (7 weeks old) was shaven under pentobarbital anethesia, the skin was cut out, and fat on the dermis side of the skin was carefully removed. The skin with the dermal side down was sticked to a vertical-type diffusion cell where 37°C water was previously circulated, and the preparation prepared according to the method described above being cut into a circular piece with 1 cm diameter was applied to the center and then fixed by sandwiching it with a dome type cell. The receiver solution maintained in a thermostatic chamber at 37°C was introduced into the dermal side, a L-tube was mounted to a sampling port and fixed with Parafilm, and thereafter, a predetermined amount of the receiver solution was further added. The receiver solution was stirred with a magnetic stirrer, and an aliquot of the receiver solution was collected periodically, and the same volume of the receiver solution was replenished. The amount of the drug in the sample collected was determined with a HPLC, and the amount of oxybutynin hydrochloride penetrated through the skin was calculated.

(HPLC conditions)

Column: Capsule Pack C18, UG 5μm, 4.6 mm x 150 mm (by Shiseido K.K.)
Column temperature: Constant temperature at around 40°C
Flow rate: Constant flow rate at around 1 ml/min
Detection wavelength: UV 230 nm
Injection volume: 10 μl
Mobile phase: 0.2% Triethylamine aqueous solution (pH 3.5):acetonitrlle=3:2

2) Result

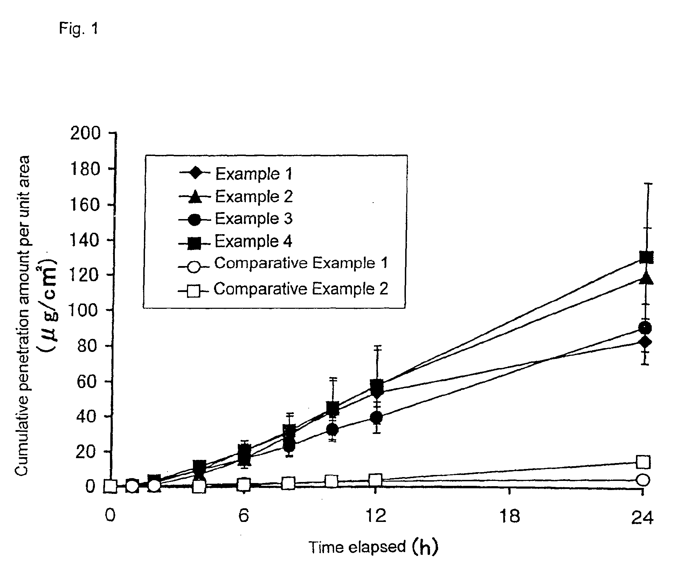

The cumulative amount of oxybutynin hydrochloride with time of each preparation is shown in figure 1. The transdermal absorption preparation containing oxybutynin hydrochloride using N-vinyl acetamide as the base according to Examples 1, 2, 3 and 4 showed sustained and yet high penetration property from the initial stage of the application. When the cumulative amounts after 24 hours were compared with the tape preparation with the rubbery type base, the preparations of Examples 1, 2, 3 and 4 each were 16.5 times, 23.7 times, 18.3 times and 26.4 times respectively. Furthermore, when they are compared with the aqueous transdermal absorption preparation with aqueous polymeric compound other than N-vinyl acetamide copolymer shown in Comparative Example 2, the preparations of Examples 1, 2, 3 and 4 each were 5.1 times, 7.8 times, 6.2 times and 8.5 times respectively. Therefore, the transdermal absorption preparations of the present invention show superior skin penetration property.

Experiment 2: Drug stability test

1) Test method

The preparations shown in Examples 2 and 4 in the sealed aluminum bags were stored in a thermostatic chamber set at 40°C for 6 months, and residual amounts of the drug in each preparation upon storage were quantitatively determined with a HPLC according to the conditions specified in Experiment 1 and calculated according to the following equation.

Residual amount of drug (%)=(drug content after 6 months at 40°C/drug content at the time of the production) x 100

2) Result

Table 1

| Drug stability test | |
| --- | --- |
| | Residual drug proportion (%) |
| Example 2 | 96.50 |
| Example 4 | 95.40 |

As shown in Table 1, both the non-aqueous preparation according to Example 2 with N-vinyl acetamide copolymer as the base and the aqueous preparation according to Example 4 showed high amount of residual drug proportion for oxybutynin and demonstrated high drug stability.

Experiment 3: Rabbit skin primary irritation test

1) Test method

Skin primary irritation test was conducted using JW/CSK male rabbit (12 weeks old) with their the back shaven on the day before the test, and was evaluated with a primary irritation index of Draize method. After the normal skin region and the damaged skin region excoriated with a sterilized injection needle were marked, the tests were conducted regarding the irritability to the respective skin regions. The applied area was set to a size of 5 cm$^2$, and one sheet each of the preparations according to Example 1 and Example 2 was applied to the normal skin region and the damaged skin region respectively. After 24 hours upon application, the preparations were removed, and the formation of erythema and edema, and incrustation were evaluated in the applied regions according to the Draize's criterion after 1 hour, 24 hours and 48 hours upon removal. From the result, skin primary irritation index (P.I.I) was calculated, and the evaluation was made according to the evaluation classification.
0.25 g of an ointment with 5% sodium lauryl sulfate-95% white vaseline (Control 1, applied by spreading out over a non-woven fabric) and the only base preparation in which oxybutynin hydrochloride having been removed from the preparation according to Example 2 (Control 2) were also evaluated as controls.

2) Result

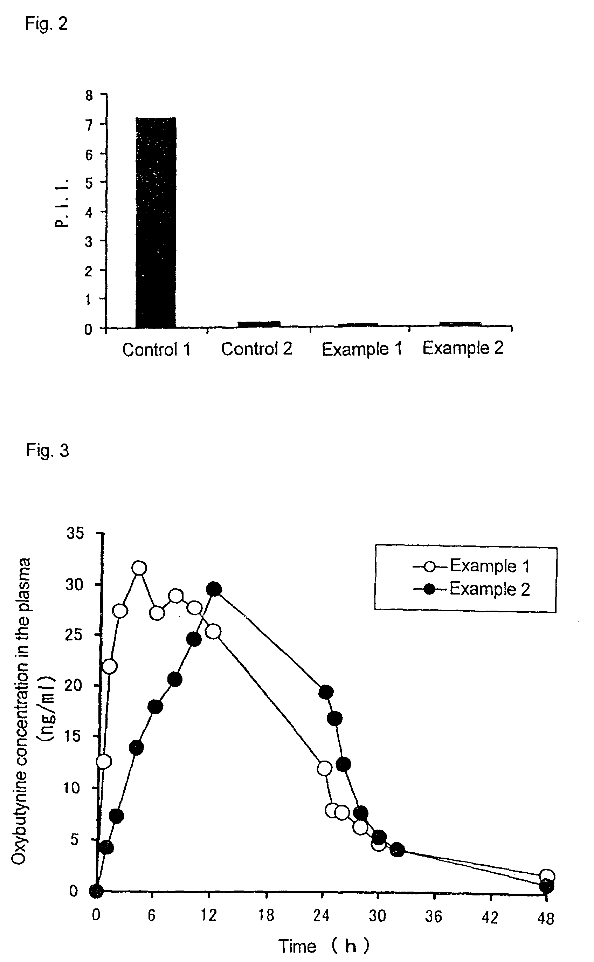

The results are shown in Fig. 2. It is apparent that the transdermal absorption preparations according to Example 1 and Example 2 as well as Control 2 (base) each fall within the weak irritant according to the evaluation classification of Draize's criterion, and are preparations with extremely low skin irritability.

Experiment 4: Oxybutynine concentration in the rabbit plasma

1) Test method

The back of JW/CSK male rabbit (12 weeks old) was shaven, and the transdermal absorption preparations of the present invention according to Example 1 and Example 2 were applied and the preparations were removed from the back after 24 hours upon application. Exsanguination was conducted from the ear vein, and the oxybutynine hydrochloride concentration in the plasma was measured with a LC/MS.

2) Result

The results are shown in Fig. 3. The preparations shown in Example 1 and Example 2 could maintain the concentration in the plasma for the long time, and moreover, the concentration in the plasma decreased rapidly upon the removal of the preparation.

Experiment 5: Influence on the cystometrogram of a vigilant rabbit

1) Test method

JW/CSK male rabbits (2.0~2.5 kg in body weight) were used as the test animals, and 5~6 rabbits as a group were tested. The back of rabbits was shaven, rabbits were fixed on the back with the supine position under etherization, and the abdominal region was shaven. Thereafter, the mesial incision was made on the abdomen, thereby exposing the bladder. A catheter for the bladder was inserted from the ureter and fixed, and one end of a polyethylene tube connected to a T-tube was connected thereto. Thereafter the abdomen was sutured. The one end of the T-tube was used as a catheter for the measurement of the cystometrogram, and other end of the T-tube was used as a catheter for pouring the physiological saline solution.

The rabbits were moved to the restraint cages, and the physiological saline solution was poured into the bladder at the puring rate of 20 ml/10 min using a continuous infusion pump (a product of Toyo Sangyo K.K.) under the vigilant condition. The changes in the internal pressure of bladder from the onset of the infusion to urination were recorded with a multi-purpose measurement recording device (RMP-6004, Nippon Koden K.K.) through a pressure transducer. After the animal was awakened, two preparations shown in Example 1 were applied on the back, and urination interval and urination pressure after 2~3, 4~5 and 6~7 hours from the application were obtained. From the ratio of the value before the application to the value after the application, the urination prolongation rate and the urination suppression rate were calculated and evaluated.

2) Result

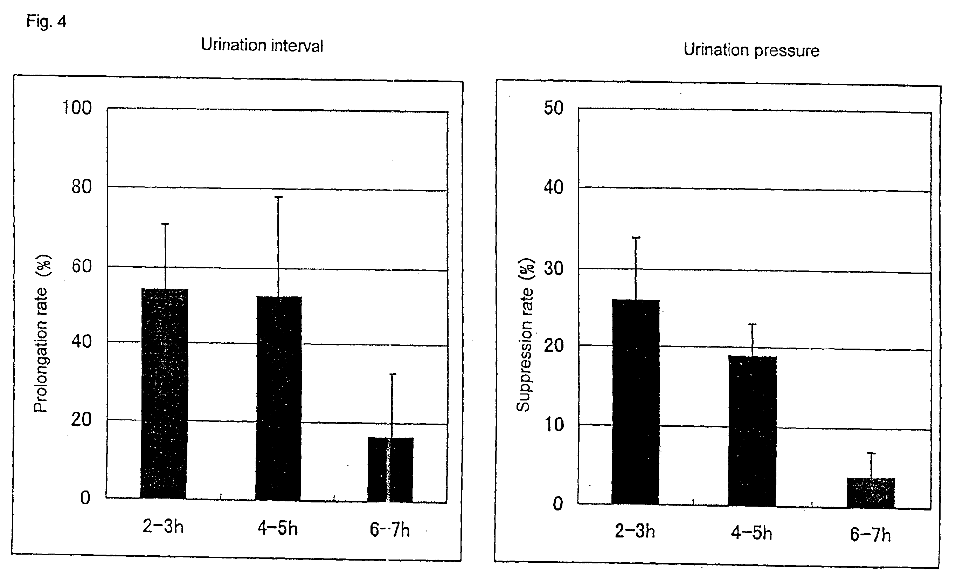

The results are shown in Fig. 4. The preparation shown in Example 1 prolonged the urination interval for 54.2% and suppressed the urination pressure for 26.0% after the application of 2~3 hours. After 4~5 hours upon application, the urination interval was prolonged for 52.6%, and the urination pressure was suppressed for 18.8%. However, after 6~7 hours upon application, the prolongation of the urination interval was decreased to 16.3%, and the suppression of the urination pressure was decreased to 3.8%.

The results described above show that the transdermal absorption preparation of the present invention is effective for the treatment of urinary incontinence and pollakiuria.

**Claims**

1. A transdermal absorption preparation containing oxybutynin comprising oxybutynin or its pharmacologically acceptable salt thereof as the active ingredient, and N-vinyl acetamide copolymer as the main components, containing N-vinyl acetamide and a polymerizable monomer having COOM group, wherein M is hydrogen atom, an alkali metal or ammonium, as a base component.

**2.** The transdermal absorption preparation containing oxybutynin according to claim 1, wherein said preparation comprises the base component containing N-vinyl acetamide copolymer with N-vinyl acetamide and a polymerizable monomer having COOM group, wherein M is hydrogen atom, an alkali metal or ammonium, as the main components, a cross-linking agent, di- or polyhydric alcohols and a stabilizer, and oxybutynin or its pharmacologically acceptable salt thereof as the active ingredient.

**3.** The transdermal absorption preparation containing oxybutynin according to claim 2, wherein di- or polyhydric alcohols are one or more than two kinds selected from the group consisting of polyethylene glycol, propylene glycol, 1,3-butanediol and glycerin.

**4.** The transdermal absorption preparation containing oxybutynin according to claim 2, wherein the stabilizer is a pH regulating agent.

**5.** The transdermal absorption preparation containing oxybutynin according to claim 2, wherein said preparation comprises 2% to 50% by weight of N-vinyl acetamide copolymer, 0.01% to 20% by weight of a cross-linking agent, 10% to 90% by weight of di- or polyhydric alcohols and 0.01% to 10% by weight of a stabilizer as the base components, and 0.01% to 30% by weight of oxybutynin or its pharmacologically acceptable salt thereof as the active ingredient, based on the total weight of the preparation.

**6.** The transdermal absorption preparation according to claim 2, wherein said base components comprise additionally a percutaneous absorption accelerator or a solvent, or both skin absorption accelerator and solvent.

**7.** The transdermal absorption preparation containing oxybutynin according to claim 6, wherein said percutaneous absorption accelerator are one or more than two kinds selected from the group consisting of propylene glycol, crotamiton, N-methyl-2-pyrrolidone, I-menthol, lactic acid esters and fatty acid esters.

**8.** The transdermal absorption preparation according to claim 6, wherein said preparation comprises 2% to 50% by weight of N-vinyl acetamide copolymer, 0.01% to 20% by weight of a cross-linking agent, 10% to 90% by weight of di- or polyhydric alcohols, 0.5% to 20% by weight of a percutaneous absorption accelerator or a solvent, or both percutaneous absorption accelerator and solvent and 0.01% to 10% by weight of a stabilizer as the base components, and 0.01% to 30% by weight of oxybutynin or its pharmacologically acceptable salt thereof as the active ingredient, based on the total weight of the preparation.

**9.** The transdermal absorption preparation containing oxybutynin according to claim 1, wherein said preparation comprises the base component containing N-vinyl acetamide copolymer with N-vinyl acetamide and a polymerizable monomer having COOM group, wherein M is hydrogen atom, an alkali metal or ammonium, as the main components, a cross-linking agent, di- or polyhydric alcohols, water, aqueous polymeric compounds and a stabilizer, and oxybutynin or its pharmacologically acceptable salt thereof as the active ingredient.

**10.** The transdermal absorption preparation containing oxybutynin according to claim 9, wherein di- or polyhydric alcohols are one or more than two kinds selected from the group consisting of polyethylene glycol, propylene glycol, 1,3-butanediol and glycerin.

**11.** The transdermal absorption preparation according to claim 9, wherein the aqueous polymeric compounds are one or more than two kinds selected from the group consisting of poly-acrylic acid, methyl cellulose, starch acrylic acid, and acrylic acid ester copolymer and its emulsion.

**12.** The transdermal absorption preparation containing oxybutynin according to claim 9, wherein the stabilizer is the pH regulating agent.

**13.** The transdermal absorption preparation according to claim 9, wherein pH of the preparation is in the range of pH 4.5 to 5.1.

**14.** The percutanelous absorption preparation containing oxybutynin according to claim 9, wherein the base components additionally contain a percutaneous absorption accelerator or a solvent other than water, or both percutaneous absorption accelerator and solvent other than water.

**15.** The transdermal absorption preparation containing oxybutynin according to claim 14, wherein the percutaneous absorption accelerator are on or more than two kinds selected from the group consisting of cetyl lactate, oleyl alcohol, Crotamiton, isopropanol, N-methyl-2-pyrrolidone or EDTA.

16. The transdermal absorption preparation containing oxybutynin according to claim 15, wherein the percutaneous absorption accelerator is oleyl alcohol.

17. The transdermal absorption preparation containing oxybutynin according to claim 14, wherein said preparation comprises 2% to 50% by weight of N-vinyl acetamide copolymer, 0.01% to 20% by weight of a cross-linking agent, 10% to 90% by weight of di- or polyhydric alcohols, 0.5% to 20% by weight of a percutaneous absorption accelerator or a solvent other than water, or both percutaneous absorption accelerator and solvent other than water, 5% to 90% by weight of water, 3% to 70% by weight of an aqueous polymeric compound, 0.01% to 10% by weight of a stabilizer as the base components, and 0.01% to 30% by weight of oxybutynin or its pharmacologically acceptable salt thereof as the active ingredient, based on the total weight of the preparation.

18. The transdermal absorption preparation containing oxybutynin according to claims 1, 2 or 9, wherein the preparation is medical plaster-type preparation.

Fig. 1

Fig. 2

Fig. 3

Fig. 4

Urination interval

Urination pressure

EP 1 174 132 A1

**INTERNATIONAL SEARCH REPORT**

| International application No. |
| --- |
| PCT/JP00/02697 |

A. CLASSIFICATION OF SUBJECT MATTER
Int.Cl$^7$   A61K31/216, 47/32, 9/70, A61P31/02

According to International Patent Classification (IPC) or to both national classification and IPC

B. FIELDS SEARCHED

Minimum documentation searched (classification system followed by classification symbols)
Int.Cl$^7$   A61K31/216, 47/32, 9/70, A61P31/02

Documentation searched other than minimum documentation to the extent that such documents are included in the fields searched

Electronic data base consulted during the international search (name of data base and, where practicable, search terms used)
CAPLUS(STN),MEDLINE(STN),EMBASE(STN)

C. DOCUMENTS CONSIDERED TO BE RELEVANT

| Category* | Citation of document, with indication, where appropriate, of the relevant passages | Relevant to claim No. |
| --- | --- | --- |
| PX | EP, 965626, A1 (Ssp Co. Ltd.),<br>22 December, 1999 (22.12.99),<br>Whole document,<br>& JP, 2000-7559, A | 1-18 |
| A | JP, 10-316590, A (Showa Denko K.K.),<br>02 December, 1998 (02.12.98)   (Family: none)<br>& Database CAPLUS on STN, AMERICAN CHEMICAL SOCIETY (ACS),<br>Columbus, OH, USA), DN.130:43316 | 1-18 |
| A | JP, 10-25243, A (Showa Denko K.K.),<br>27 January, 1998 (27.01.98)   (Family: none)<br>& Database CAPLUS on STN, AMERICAN CHEMICAL SOCIETY (ACS),<br>Columbus, OH, USA), DN.128:184711 | 1-18 |
| A | JP, 9-176015, A (Showa Denko K.K.),<br>08 July, 1997 (08.07.97)   (Family: none)<br>& Database CAPLUS on STN, AMERICAN CHEMICAL SOCIETY (ACS),<br>Columbus, OH, USA), DN.127:181152 | 1-18 |
| A | JP, 9-143061, A (Showa Denko K.K.),<br>03 June, 1997 (03.06.97)   (Family: none) | 1-18 |

☒ Further documents are listed in the continuation of Box C.          ☐ See patent family annex.

| * | Special categories of cited documents: |
| --- | --- |
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 18 July, 2000 (18.07.00) | 01 August, 2000 (01.08.00) |

| Name and mailing address of the ISA/ | Authorized officer |
| --- | --- |
| Japanese Patent Office |  |
| Facsimile No. | Telephone No. |

Form PCT/ISA/210 (second sheet) (July 1992)

## INTERNATIONAL SEARCH REPORT

| | International application No. |
|---|---|
| | PCT/JP00/02697 |

C (Continuation). DOCUMENTS CONSIDERED TO BE RELEVANT

| Category* | Citation of document, with indication, where appropriate, of the relevant passages | Relevant to claim No. |
|---|---|---|
| | & Database CAPLUS on STN, AMERICAN CHEMICAL SOCIETY (ACS), Columbus, OH, USA),DN.127:99853 | |
| A | EP, 446636, A1 (Showa Denko K.K.), 18 September, 1991 (18.09.91) & US, 5254338, A    & US, 5344655, A & US, 5455042, A    & JP, 4-182437, A | 1-18 |
| A | JP, 4-266821, A (Rido Chemical Co. Ltd.) 22 September, 1992 (22.09.92) (Family: none) & Databese CALPIS on STN, AMERICAN CHEMICAL SOCIETY (ACS), Columbus, OH, USA), DN.118:27492 | 1-18 |

Form PCT/ISA/210 (continuation of second sheet) (July 1992)